# EUROPÄISCHE PATENTANMELDUNG

(11) **EP 2 856 871 A1**
(43) Veröffentlichungstag der Anmeldung: **08.04.2015**
(21) Anmeldenummer: 13187164.2
(22) Anmeldetag: 02.10.2013
(51) Int. Cl.: A01N 25/10, A01N 59/20, A61L 2/232, A61L 2/238

(54) **Antimikrobiell ausgerüstetes Formteil auf Silikonbasis**

(71) Anmelder: Cu Innotech GmbH, 63477 Maintal (DE)
(72) Erfinder: Frontzek, Christian, Amadeus, 63452 Hanau (DE)
(74) Vertreter: Walkenhorst, Andreas

(57) **Zusammenfassung**

Ein Formteil (1,1') mit einem mikrobizid ausgebildeten Berührungsbereich (2,2') auf Kupferbasis soll eine besonders hohe Vielfalt an möglichen Anwendungen bei zuverlässig hoher mikrobizider Wirksamkeit in seinem Berührungsbereich (2,2') ermöglichen. Dazu weist der Berührungsbereich (2,2') erfindungsgemäß eine mikrobizide, aus in eine Silikonmatrix (8) eingebetteten Kupfer-Partikeln (10) gebildete Oberflächenschicht (6) auf.

## Beschreibung

Die Erfindung betrifft ein Formteil mit einem mikrobizid ausgebildeten Berührungsbereich. Sie bezieht sich weiter auf ein Verfahren zur Herstellung eines derartigen Formteils.

Im Alltag kommen Menschen häufig unbewusst mit gesundheitsschädlichen Bakterien, Keimen und Viren in Berührung. Besonders dort, wo man mit Flächen in Kontakt kommt, die von vielen anderen Menschen zuvor berührt worden sind, kann eine zunehmende Ansammlung von Keimen vermehrt zu unhygienischen Bedingungen führen. Um dem entgegenzuwirken, können derartige Gegenstände mikrobizid in dem Sinne ausgerüstet werden, dass Oberflächen oder Oberflächenbereiche keim- oder bakterientötend ausgeführt sind. Zu diesem Zweck kann beispielsweise ein Desinfektionselement mit einem entsprechend mikrobizid oder keimtötend ausgeführten Berührungsbereich vorgesehen sein. Einem derartigen Formteil liegt die Auslegung zugrunde, dass in dem zur Berührung vorgesehenen Berührungsbereich, beispielsweise der Oberfläche eines Griffs, einer Drückertaste oder dergleichen, die äußere oder freiliegende und damit der Berührung ausgesetzte Oberfläche mit einem geeignet gewählten mikrobiziden Material versehen wird, so dass die sich in Folge der wiederkehrenden Berührungen ansammelnden Keime oder Mikroorganismen unmittelbar wieder abgetötet werden und sich damit bereits von vornherein nicht vollständig ansiedeln können.

Ein derartiges Desinfektionselement mit einem mikrobizid ausgeführten Berührungsbereich ist beispielsweise aus der DE 10 2009 013 029 A1 bekannt. Dieses bekannte Desinfektionselement basiert von seiner Auslegung her auf der Nutzung von Kupfer als Basismaterial für die angestrebte mikrobizide Wirkung.

Diese Nutzung von Kupfer wird auch vorliegend als besonders vorteilhaft angesehen, da gerade Kupfer eine Vielzahl von für diesen Einsatzzweck besonders günstigen Eigenschaften aufweist. Einerseits zeigt nämlich Kupfer als Material für viele Mikroorganismen schon in geringen Mengen eine toxische Wirkung und kann somit besonders zuverlässig zur Abtötung von Keimen oder Mikroorganismen verwendet werden. Andererseits ist Kupfer aber - im Gegensatz beispielsweise zu Silber oder anderen mikrobiziden Grundstoffen - Bestandteil des natürlichen menschlichen Stoffwechsels und daher grundsätzlich für den menschlichen Organismus unschädlich.

Eine Kontamination des menschlichen Organismus durch ein Desinfektionselement auf Kupferbasis ist daher selbst bei hoher mikrobizider Wirkung nicht zu befürchten. Die nachfolgenden Ausführungen beziehen sich daher auf die als besonders vorteilhaft angesehene Verwendung von Kupfer als mikrobizider Basisstoff. In analoger und ebenfalls als im Sinne der vorliegenden Erfindung angesehene Ausgestaltung kann in denjenigen Fällen, bei denen eine besonders gute Verträglichkeit mit dem menschlichen Organismus kein Auslegungsziel mit hoher Priorität darstellt, anstelle von Kupfer grundsätzlich auch ein anderer geeigneter mikrobizider Grundstoff wie beispielsweise Silber, Zink oder dergleichen oder auch eine Mischung dieser Materialien vorgesehen sein.

Die genannten Eigenschaften von Kupfer als mikrobizider Basisstoff werden beim Desinfektionselement nach der DE 10 2009 013 029 A1 gezielt genutzt. Bei diesem bekannten Desinfektionselement ist zudem gezielt dem Umstand Rechnung getragen, dass für eine ausreichend hohe mikrobizide oder keimtötende Wirkung ein Mindestmaß der Anzahl der pro Flächen- und Zeiteinheit an die mit dem Berührungsbereich in Kontakt tretenden Gewebeflächen abgegebenen Kupferionen, nachfolgend auch als "lonenausschüttung" bezeichnet, eingehalten werden sollte.

Das aus der DE 10 2009 013 029 A1 bekannte Desinfektionselement ist in diesem Sinne für eine besonders hohe lonenauschüttung im Berührungsbereich ausgelegt und umfasst zu diesem Zweck im Berührungsbereich eine Oberflächenstruktur auf Kupferbasis, die eine im Vergleich zur äußeren Oberfläche vergrößerte innere Oberfläche aufweist. Diese Auslegung des bekannten Desinfektionselements setzt allerdings eine vergleichsweise massive Bauweise des Berührungsbereichs in seiner frei liegenden Oberfläche voraus. Das bekannte Desinfektionselement ist daher in seinen möglichen Anwendungsbereichen nur begrenzt flexibel.

Der Erfindung liegt daher die Aufgabe zugrunde, ein Formteil der oben genannten Art anzugeben, das selbst bei zuverlässig hoher mikrobizider Wirksamkeit in seinem Berührungsbereich eine besonders hohe Vielfalt an möglichen Anwendungen und insbesondere eine besonders hohe Flexibilität bei seiner Formgebung ermöglicht. Des Weiteren soll ein zur Herstellung eines derartigen Formteils besonders geeignetes Verfahren angegeben werden.

Bezüglich des Formteils wird diese Aufgabe erfindungsgemäß gelöst, indem es in seinem Berührungsbereich eine mikrobizide, aus in eine Silikonmatrix eingebetteten Kupfer-Partikeln gebildete Oberflächenschicht aufweist.

Unter "Silikonmatrix" ist hierbei ein Trägermaterial auf Silikonbasis, also beispielsweise ein Silikonkautschuk, ein Silikonelastomer, ein Silikonharz, ein Fluorsilikon oder dergleichen zu verstehen, in das die für die Bereitstellung der mikrobiziden Eigenschaften vorgesehene Kupfer in Form der Kupferpartikel eingebettet ist.

Die Erfindung geht von der Überlegung aus, dass die genannten besonders günstigen mikrobiziden Eigenschaften von Kupfer auch über die bisher vorgesehenen Einsatzbereiche als Desinfektionslement im eigentlichen Sinne auch für andere Einsatzzwecke nutzbar gemacht werden können, indem dieses unverändert als Basisstoff in ein geeignet gewähltes, für eine besonders große Bandbreite an Einsatzgebieten geeignetes Matrixmaterial eingebunden wird. Um dabei eine besonders hohe Flexibilität beim Einsatz und der Ausgestaltung des Formteils unter Erhalt der mikrobiziden Eigenschaften der Kupferpartikel zu ermöglichen, ist als Matrixmaterial ein Material auf Silikonbasis vorgesehen. Wie sich nämlich völlig überraschend herausgestellt hat, ist die Einbettung von Kupferpartiekln in eine Silikonmatrix auch unter Erhalt der mikrobiziden Eigenschaften der Kupferpartikel möglich, wobei das Matrixmaterial selbst nach der Ausrüstung mit den Kupferpartikeln auf besonders einfache Weise einer Formgebung unterzogen werden kann. Auf diese Weise ist eine zuverlässige Ausrüstung auch vergleichsweise komplexer Geometrien mit einer mikrobiziden Oberflächenschicht und damit eine besonders große Vielzahl an möglichen räumlichen Ausgestaltungen des Formteils oder von dessen Berührungsbereich möglich.

Als Basismaterial zur Bildung der Silikonmatrix kann dabei grundsätzlich jedes geeignete Silikon vorgesehen sein. Gerade im Hinblick auf die angestrebte besonders hohe Flexibilität bei der Formgebung ist aber besonders bervorzugt als Basismaterial zur Bildung der Silikonmatrix Flüssigksilikon, ein Silikonkautschuk oder ein Silikonelastomer vorgesehen. Unter einem Silikonkautschuk ist dabei eine in den gummielastischen Zustand überführbare Masse zu verstehen, die Poly(organo)siloxane enthält, die ihrerseits für Vernetzungsreaktionen zugängliche Gruppen aufweisen. Als solche kommen vorwiegend Wasserstoffatome, Hydroxygruppen und Vinylgruppen in Frage, die sich an den Kettenenden befinden, aber auch in die Kette eingebaut sein können. Silikonkautschuke enthalten verstärkende Stoffe und Füllstoffe, deren Art und Menge das mechanische und chemische Verhalten der durch die Vernetzung entstehenden Silikonelastomere deutlich beeinflussen. Silikonkautschuke können darüber hinaus mit geeigneten Pigmenten gefärbt werden. Durch die Zusatzstoffe ist dabei - auch bei einer Einlagerung der Kupferpartikel - durch eine geeignete Wahl und Kombination der Basisstoffe selbst bei Erhalt der durch die Kupferpartikel bedingten mikrobiziden Eigenschaften eine große Vielfalt hinsichtlich Form und mechanischer Eigenschaften des mikrobizid ausgerüsteten Formkörpers erreichbar.

Bei der Klassifizierung der Silikonkautschuke unterscheidet man nach der notwendigen Vernetzungstemperatur zwischen kalt- (RTV) und heißvernetzenden (HTV) Silikonkautschuken (RTV = raumtemperatur vernetzend, HTV = hochtemperatur vernetzend). HTV-Silikon-Kautschuke sind plastisch verformbare Materialien. Sie enthalten sehr oft organische Peroxide für die Vernetzung. Die daraus durch die Vernetzung bei hoher Temperatur hergestellten Elastomere sind wärmebeständige, zwischen -40 und 250 °C elastische Produkte, die z. B. als hochwertige Dichtungs-, Dämpfungs-, Elektroisolierbauteile, Kabelummantelungen und dergleichen verwendet werden. Besonders vorteilhaft ist die mikrobizide Ausrüstung gerade derartiger Formteile durch die Einbettung der Kupferpartikel, da damit zusätzlich zu dem eigentlich vorgesehenen Einsatzzweck des Formteils (beispielsweise als Dichtung) die errreichbaren mikrobiziden Eigenschaften als Zusatznutzen verfügbar gemacht werden können.

Ein anderer Vernetzungsmechanismus bei der Herstellung eines Silikonelastomers besteht in einer meist durch Edelmetallverbindungen katalysierten Addition von Si-H-Gruppen an siliciumgebundene Vinylgruppen, die beide in die Polymerketten bzw. an deren Ende eingebaut sind. Die dabei eingesetzten Silikonkautschuk-Komponenten, die im Unterschied zu den oben beschriebenen HTV-Kautschuken eine niedrigere Viskosität aufweisen und somit pumpbar sind, werden mit geeigneten Misch- und Dosiermaschinen dosiert, gemischt und meistens in Spritzgießautomaten verarbeitet, so dass wiederum eine besonders hohe Freiheit und Flexibilität bei der Formgebung von solchermaßen hergestellten Formteilen erreichbar ist. Wie sich überraschenderweise herausgestellt hat, sind dabei besonders günstige mikrobizide Eigenschaften durch die Einlagerung der Kupferpartikel in derartiges Matrixmaterial erreichbar, indem in ganz besonders vorteilhafter Ausgestaltung Platin oder eine platinhaltige Verbindung als Katalysatormaterial für den Vernetzungsmechanismus bei der Herstellung des Silikonelastomers verwendet wird.

Bei den RTV-Silikonkautschuken lassen sich Ein- und Zweikomponentensysteme unterscheiden. Die erste Gruppe (RTV-1) vernetzt bei Raumtemperatur unter dem Einfluss von Luftfeuchtigkeit, wobei die Vernetzung durch Kondensation von SiOH-Gruppen unter Bildung von Si-O-Bindungen erfolgt. Die SiOH-Gruppen werden durch Hydrolyse von SiX-Gruppen einer intermediär aus einem Polymer mit endständigen OH-Gruppen und einem sogenannten Vernetzer R-SiX3 (X = -O-CO-CH3, -NHR) entstehenden Spezies gebildet. Bei Zweikomponentenkautschuken (RTV-2) werden als Vernetzer z. B. Gemische aus Kieselsäureestern (z. B. Ethylsilicat) und zinnorganische Verbindungen verwendet, wobei als Vernetzungsreaktion die Bildung einer Si-O-Si-Brücke aus Si-OR und Si-OH durch Alkoholabspaltung erfolgt.

Die mikrobizide Oberflächenschicht des Berührungsbereichs kann grundsätzlich als auf einen Tragkörper aufgebrachte Beschichtung ausgeführt sein. Damit ist eine besonders weitgehende Flexibilität insbesondere hinsichtlich möglicher Geometrieformen erreichbar. Die Einbindung der Kupferpartikel in das Matrixmaterial ermöglicht dabei eine besonders flexible Anwendung des mikrobiziden Materials auch an verschiedenartig geformten oder ausgestalteten Flächenbereichen des Tragkörpers. In diesem Sinne bildet das Formteil somit seinerseits eine an den eigentlichen Tragkörper angebrachte Bschichtung.

In besonders vorteilhafter Ausgestaltung ist das Formteil aber in der Art eines (vorzugsweise elastischen) Festkörpers als Vollkörper ausgeführt, der vollständig von dem ausgehärteten, mit den eingebetteten Kupfer-Partikeln versehenen Matrixmaterial auf Silikonbasis gebildet wird. Dabei kann das Formteil beispielsweise in der Art eines Gusskörpers hergestellt sein, wobei bei der Herstellung auf für das jeweils vorgesehene Silikon geeignet gewählte Verfahren zurückgegriffen werden kann. Dabei kann das solchermaßen gebildete Formteil mit einer zumindest annähernd räumlich homogenen, gleichmäßigen Verteilung der eingebetteten Kupfer-Partikel versehen sein, bei der sämtliche Raumbereiche des Formteils eine vergleichbare Konzentration an eingebetteten Kupfer-Partikeln aufweisen. In besonders vorteilhafter Ausgestaltung kann die Konzentration der eingebetteten Kupfer-Partikel aber auch räumlich inhomogen und im oberflächennahen Bereich überhöht sein, so dass der überwiegende Anteil der Kupfer-Partikel nah zur Oberfläche des Berührungsbereichs positioniert ist.

Ein derartiger, gezielt gewählter Konzentrationsverlauf der Kupfer-Partikel ist beispielsweise erhältlich, indem bei einer Verwendung eines Flüssig-Silikon-Grundstoffs die Kupfer-Partikel während des Herstellungsprozesses des Silikonkörpers dem zur Bildung der Silikonmatrix vorgesehenen Ausgangsmaterial beigemischt und in diesem vorübergehend in Suspension oder Schwebe gehalten werden. Während des Aushärtens des Ausgangsmaterials zur Bildung der Matrix können die Kupfer-Partikel schwerkraftbedingt zu Boden sinken, so dass eine Anreicherung der Partikel im unteren Grenzflächenbereich des entstehenden Gusskörpers erfolgt. Dieser untere Grenzflächenbereich kann sodann zur Bildung des Berührungsbereichs herangezogen werden.

Die Verteilung der Partikel im Gusskörper kann dabei durch geeignete Parameterwahl bei der Herstellung besonders bedarfsgerecht eingestellt werden. Insbesondere durch eine geeignete Wahl der Prozesszeiten, also insbesondere der für die Aufkonzentration der Partikel im Grenzflächenbereich gewählten Zeit, im Hinblick auf die Stoffeigenschaften des zur Bildung der Matrix vorgesehenen Ausgangsmaterials, insbesondere dessen Viskosität, kann dabei eine gewünschte Konzentration der Partikel im oberflächennahen Bereich zuverlässig und reproduzierbar eingestellt werden.

Bei der Einbettung der Kupferpartikel in das Matrixmaterial wird vorteilhafterweise in besonderem Maße berücksichtigt, dass zur frei liegenden Außenoberfläche des Berührungsbereichs hin eine ausreichend hohe lonenausschüttung an Kupferionen stattfindet, so dass die mikrobizide Wirkung zuverlässig aufrechterhalten werden kann. Dies wird einerseits durch die oben genannte Aufkonzentration von Kupfer-Partikeln in Oberflächennähe begünstigt. Wie sich überraschenderweise herausgestellt hat, ist dies andererseits aber auch auf besonders günstige Weise erreichbar, indem in vorteilhafter Ausgestaltung die Kupferpartikel in der Matrix als so genannte Nano- oder Mikropartikel, also als Partikel mit einer durchschnittlichen Größe im Nano- oder Mikrometerbereich, vorliegen.

In besonders vorteilhafter Ausgestaltung umfassen somit die in die Matrix eingebetteten Kupfer-Partikel eine Partikelfraktion mit einer Partikelgröße von höchstens 500 nm, besonders bevorzugt mit einer Partikelgröße von 90 bis 250 nm, und/oder eine Partikelfraktion mit einer Partikelgröße von mindestens 1 µm. Die Partikelfraktion weist dabei besonders bevorzugt eine mittlere Partikelgröße von etwa 20 µm bis 50 µm und/oder eine Standardabweichung von höchstens 20% auf. Durch eine derartige Wahl der Partikelgröße im Nano- oder Mikrometerbereich ist unter anderem sichergestellt, dass die Partikel ein besonders großes Oberflächen-Volumen-Verhältnis und damit eine besonders große spezifische Oberfläche aufweisen, was die lonenabgabe sehr begünstigt.

Hinsichtlich ihrer räumlichen Gestalt können die Kupferpartikel als Partikel mit kugeliger oder zumindest näherungsweise runder Partikelform ausgeführt sein. In alternativer oder zusätzlicher besonders vorteilhafter Ausgestaltung sind die Kupferpartikel aber auch in ihrer Partikelform gezielt im Hinblick auf eine besonders hohe lonenausschüttung bei vergleichsweise gering gehaltenem Materialeinsatz oder-aufwand gewählt. Wie sich völlig überraschend herausgestellt hat, ist dies besonders vorteilhaft erreichbar, indem die Kupferpartikel als flocken- oder blättchenförmige (Mikro)Partikel ausgeführt. Derartige blättchenförmig ausgeführte Kupferpartikel sind beispielsweise unter dem Handelsnamen "Cubrotec 6000" erhältlich. Diese weisen bei lamellarer Kornform und einem mittleren Teilchendurchmesser von etwa 35 µm einen Kupfergehalt von etwa 98% und eine Fülldichte von etwa 1g/cm³ auf. Im Gegensatz dazu weist Kupferpulver mit kugeliger Partikelform eine Fülldichte von etwa 5g/cm³ auf, so dass durch die Partikel mit lamellarer Kornform ein um den Faktor 5 geringerer Materialeinsatz erforderlich ist. Dennoch konnte mit dieser besonders bevorzugten Kornform bei gleichem Volumenanteil die gleiche antimikrobielle Wirksamkeit erzielt werden. Bei der Herstellung wird das blättchenförmige Kupferpulver besonders bevorzugt zunächst luftverdüst und anschließend in einer Kugelmühle platt gemahlen.

Im Hinblick auf eine besonders günstige Verarbeitbarkeit und Applizierbarkeit des vorgesehenen kupferhaltigen Mikrobizids wird das zur Bildung der Silikonmatrix vorgesehene Material zweckmäßigerweise gezielt ausgewählt. Dabei sollte als Auslegungsparameter unter anderem einerseits die Fähigkeit berücksichtigt werden, die Kupferpartikel zuverlässig und gff. auch gleichmäßig zu dispergieren, so dass möglichst keine oder nur geringe Agglomeration stattfindet. Andererseits sollte aber auch eine besonders gute Verarbeitbarkeit bei der Formgebung, also bei der Herstellung eines Gusskörpers und/oder bei der Aufbringung der Beschichtung, angestrebt werden. Dazu ist insbesondere das Vorliegen eines Ausgangsmaterials als Flüssigkeit, in die die Kupferpartikel eingebracht werden können und die anschließend nach der Formgebung oder Beschichtung ausgehärtet werden kann, wünschenswert. Im Hinblick auf diese Kriterien ist zur Bildung der Matrix vorteilhafterweise HTV-Silikon, welches in der Herstellung durch die Einstellung der Parameter unterschiedliche Verteilungsgrade ermöglicht, oder RTV-2-Silikonkautschuk vorgesehen.

Die auslegungsbedingt vorgesehene und auch erwünschte ausreichend hohe Ionenausschüttung oder Ionenabgabe könnte einerseits dadurch sichergestellt werden, dass als Matrixmaterial vorteilhafterweise ein Material gewählt wird, das gerade für Kupferionen eine besonders hohe intrinsische Durchlässigkeit aufweist und somit eine lonenwanderung von Kupferionen möglichst nur geringfügig behindert. Alternativ oder zusätzlich wird die lonenabgabe aber auch dadurch begünstigt, dass in besonders vorteilhafter Ausgestaltung der Berührungsbereich des Formteils derart ausgeführt ist, dass die Kupfer-Partikel im Oberflächenbereich zumindest teilweise unbedeckt vom Matrixmaterial vorliegen, so dass gegebenenfalls ein direkter mechanischer Kontakt der Partikel mit sich ansiedelnden Keimen ermöglicht ist.

Wie sich nämlich völlig überraschend herausgestellt hat, ist neben der lonenabgabe der direkte Kontakt der Keime mit Kupfer oder den Kupferpartikeln besonders günstig für die antimikrobielle Aktivität. Gerade durch die oben genannten Partikelgrößen ist ein als besonders günstig angesehener Kompromiß zwischen hoher lonenabgabe und größtmöglicher fei liegender Kontaktfläche an der Oberfläche im Berührungsbereich des Formteils erreichbar. Es wird vermutet, dass offene oder freiliegende Kupferflächen an der Kontaktoberfläche eine Schädigung der Zellhülle der Mikroben bewirken und so das Eindringen der Kupferionen in das Zellinnere besonders begünstigen; demzufolge kann in alternativer besonders vorteilhafter Ausgestaltung auch ein anderes Material als Kontaktmaterial für die Mikroben gewählt werden, das ebenfalls eine derartige gezielte Schwächung der Zellhülle bewirkt.

Die Bereitstellung derartiger unbedeckter oder frei liegender Kupferflächen kann bei geeigneter Wahl der Dimensionierungsparameter (insbesondere Partikelgröße in Kombination mit der Dicke der Beschichtung) erreicht werden, indem die Kupfer-Partikel vorteilhafterweise zumindest teilweise nach oben hin unbedeckt aus dem Matrixmaterial herausragen. In alternativer oder zusätzlicher vorteilhafter Ausgestaltung kann auch ein Aufrauhungs- und/oder Ätzprozeß der Silikonmatrix vorgesehen sein, mit dem das Matrixmaterial oberhalb der eingebundenen Kupfer-Partikel zumindest teilweise entfernt und deren Oberfläche somit zumindest teilweise freigelegt wird. Dies erfolgt besonders bevorzugt durch ein mechanisches Verfahren, beispielsweise Schleifen. Die mikrobizide Wirkung eines derartig hergestellten Materialverbundes mit frei liegendem Kupferanteil an der Oberfläche hat insbesondere den Vorteil, dass insofern keinerlei Behinderung der lonenabgabe oder lonenausschüttung oder des direkten Kontakts von Zellwand und Kupferfläche durch das Matrixmaterial vorliegt.

Für die gewünschte ausreichend hohe lonenausschüttung und/oder den frei liegenden Kontaktbereich ist dabei besonders vorteilhaft vorgesehen, dass der durch die frei liegenden Kupfer-Partikel gebildete Anteil der Gesamtoberfläche der Beschichtung mindestens 10 % beträgt.

Vorteilhafterweise ist bei der Ausgestaltung des Desinfektionselements zusätzlich zu dessen Wirksamkeit (erreichbar durch eine ausreichend hohe lonenauschüttung oderabgabe und/oder durch möglichst und weitgehend direkten mechanischen Kontakt des partiell eingebetteten Kupfers zu den sich ansiedelnden Keimen) auch noch dessen mechanische Belastbarkeit und damit Langzeitstabilität selbst bei vorliegendem Abrieb oder sonstiger mechanischer Beanspruchung besonders berücksichtigt. Hierzu ist die Silikonmatrix im Hinblick auf die für die Herstellung und Konfigurierung der Silikone wählbaren Parameter bevorzugt geeignet ausgestaltet. Im Sinne einer ausreichend hohen mechanischen Stabilität sollte das Matrixmaterial, das in der Art eines Bindemittels die mechanische Stabilität unter den Kupfer-Partikeln herstellt, in zusätzlicher zweckmäßiger Ausgestaltung im Bereich der Oberflächenschicht einen geeignet gewählten Volumenanteil einnehmen. Um dem Rechnung zu tragen, beträgt vorteilhafterweise der Volumenanteil der Kupfer-Partikel am Gesamtvolumen der Oberflächenschicht zwischen 10% und 80%.

Zweckmäßigerweise weist die den Berührungsbereich des Formteils bildende, mit den Kupferpartikelns versehene Oberflächenschicht eine Schichtdicke von mindestens 1 µm, insbesondere von mindestens 10 µm, und von höchstens 100 µm auf. Diese Abmessungen sind besonders günstig, da man einerseits eine gewisse Mindestanzahl an Kupfer-Partikeln für die Abtötung der Mikroorganismen oder Keime benötigt.

Andererseits sollte die Dicke aber gerade im Hinblick auf die vorgesehene Teilchengröße der Kupfer-Mikropartikel nicht zu groß gewählt sein, damit auf besonders einfache Weise zumindest ein Teil der Partikeloberfläche vom Matrixmaterial unbedeckt gehalten werden kann.

Durch die vorgesehene Einbettung der als mikrobizides Basismaterial vorgesehenen Kupferpartikel in das eine Vielzahl von Anwendungsmöglichkeiten eröffnende Silikon als Basismaterial zur Bildung der Matrix können eine Vielzahl funktioneller Bau- oder Formteile zusätzlich zu ihrer eigentlichen Funktion in der Art eines Zusatznutzens mikrobizid ausgerüstet werden. Vorteilhafterweise wird das Formteil daher als Dichtungs-, Dämpfungs-, Elektroisolierbauteil, Kabelummantelung, O-Ring, Türdichtungsprofil, hohler nachgebender Abdichtstreifen beispielsweise für einen Kühlschrank oder eine Gefriertruhe, Schlauch, als Oberflächenbeschichtung (insbesondere für Ablagen, Medizingeräte, in Abflusssystemen, Tische, Sportgeräte), Beatmungsgerät, zur Beschichtung von Griffen, zur Beschichtung von textilem Gewebe, als Dichtung in Armaturen, Zubehör für Duschköpfe, Schutzhülle für Handys, Tablets, als griffiger Überzug für Hanteln und Trainingsgeräte, als Schuheinlage - ggf. auch in Verbindung mit anderem Material -, Matratzenauflage oder Zusatz dafür (Dekubitusprophylaxe, allergikergerecht), als Auflage für Operationstische zur Druckentlastung, als Tauchermaske oder Taucheranzug, oder besonders bevorzugt in den Anwendungsgebieten Medizintechnik, Autoindustrie, Sanitärbereich, Haushaltsgeräte, öffentlicher Raum verwendet.

Bezüglich des Herstellungsverfahrens für das Formteil wird die genannte Aufgabe gelöst, indem Kupfer-Partikel in eine Lösung eines Matrixbildners eingerührt werden und die dadurch erhaltene Suspension oder Dispersion anschließend homogenisiert wird. Dieses Ausgangsmaterial kann sodann einer Formgebung unterzogen und anschließend ausgehärtet werden, so dass ein zur Bildung des Formteils oder von dessen Berührungsbereich vorgesehener Gusskörper entsteht. Alternativ kann ein Trägerkörper zumindest teilweise mit einer mit den Kupfer-Partikeln und dem Matrixbildner versehenen Beschichtungslösung beschichtet werden.

Um die mikrobizide Wirksamkeit im hergestellten Oberflächenbereich zuverlässig sicherzustellen und insbesondere eine ausreichend hohe Ionenausschüttung, ggf. verbunden mit einer großen Kontaktfläche, zu ermöglichen, werden die Kupfer-Partikel vorteilhafterweise vor dem Einbringen in die Lösung des Matrixbildners stabilisiert, so dass Agglomeration der Partikel weitgehend vermieden wird und eine besonders große äußere Oberfläche der Kupfer-Partikel-Fraktion entsteht. Dabei und insbesondere bei Verwendung eines Flüssig-Silikons als Ausgangsstoff werden die Partikel vorzugsweise durch eine Ultraschallbehandlung in einer Flüssigkeit, vorzugsweise Ethanol, dispergiert, so dass sie weitgehend als einzelne Partikel getrennt vorliegen. Zusätzlich kann bei einem HTV-Silikon bei der Herstellung (während der Aushärt- oder Vernetzungsphase) die Verweildauer in der Heizstraße so gewählt werden, dass es zu einer Vernetzung der Silikonmoleküle mit der gewünschten Verteilungsdichte der Kupferpartikel kommt. Danach wird die Oberfläche der Kupfer-Partikel vorzugsweise durch eine geeignet gewählte chemische Komponente, bevorzugt Polyvinylpyrrolidon (PVP), funktionalisiert, so dass ein Verklumpen oder eine Agglomeration verhindert wird (so genannte sterische Stabilisierung).

Die mit der Erfindung erzielten Vorteile bestehen insbesondere darin, dass durch die Ausrüstung der Silikonmatrix mit eingebetteten Kupfer-Partikeln ein vergleichsweise gut und flexibel verarbeitbares Material bereitgestellt werden kann, das eine antimikrobielle oder mikrobizide Wirkung aufweist und somit besonders günstig und zuverlässig zu Desinfektionszwecken genutzt werden kann. Die besonders hohe Flexibilität gerade bei der Endverarbeitung durch geeignete Formgebung oder auch durch Aufbringen in Form einer Beschichtung ermöglicht dabei eine große Vielzahl an denkbaren Anwendungsmöglichkeiten und -gebieten.

Durch die Einbettung der Kupfer-Partikel, insbesondere Kupfer-Nano- oder Mikropartikel, in ein Matrixmaterial kann eine hohe Kupfer-lonenausschüttung auch für Berührungsbereiche mit vergleichsweise komplexer Geometrie oder auch in großer Stückzahl sichergestellt werden. Damit ist die zuverlässige Bereitstellung eines mikrobiziden Berührungsbereichs auch bei flexiblen oder variierenden Anforderungen auf besonders einfache Weise ermöglicht.

Ein Ausführungsbeispiel der Erfindung wird anhand einer Zeichnung näher erläutert. Darin zeigen:
- Fig. 1: ein zur Verwendung als Desinfektionselement vorgesehenes Formteil in Schnittdarstellung, und
- Fig. 2 - 4: jeweils eine alternative Ausführungsform eines Formteils ebenfalls im Schnitt.

Gleiche Teile sind in allen Figuren mit denselben Bezugszeichen versehen.

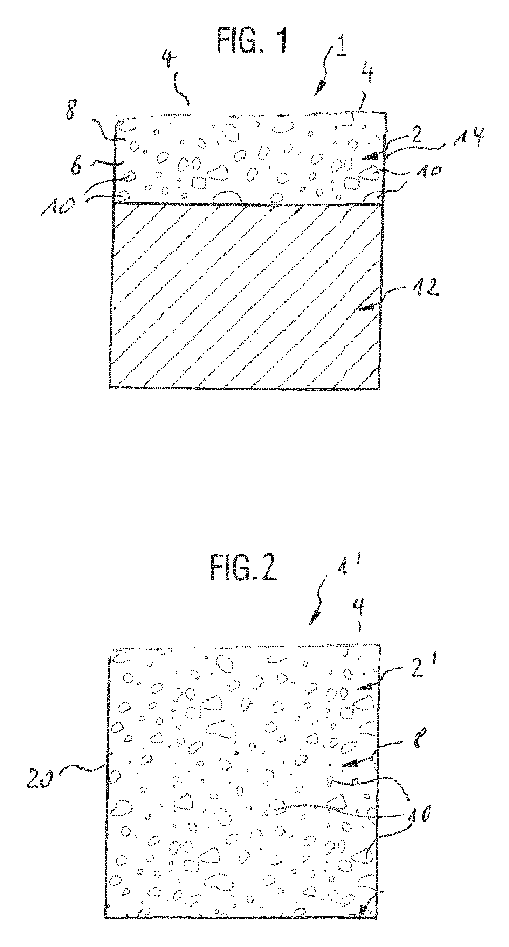

Das Formteil 1 gem. Fig. 1 ist zur Verwendung an oder in Gegenständen oder allgemein Oberflächen vorgesehen, bei denen mit Keimbelastungen oder allgemein Belastungen durch Mikroben zu rechnen ist, wie beispielsweise Dichtungen, Türgriffen oder Geländern in öffentlichen Gebäuden, oder bei denen aus sonstigen Gründen auf ein besonders hohes Maß an Keimfreiheit oder Sterilität zu achten ist, wie beispielsweise in Krankenhäusern.

Um dabei einer Verkeimung oder Verunreinigung durch Mikroorganismen gezielt und konsequent entgegenzuwirken, ist das Formteil 1 in einem Berührungsbereich 2 mikrobizid oder keimtötend ausgeführt. Der Berührungsbereich 2 ist dabei in seinem Oberflächenbereich unter Verwendung von Kupfer als mikrobizides Basismaterial ausgeführt, um die für diesen Zweck äußerst günstigen Eigenschaften von Kupfer gezielt nutzen zu können. Insbesondere soll dabei gezielt genutzt werden, dass Kupfer einerseits für viele Mikroorganismen schon in geringen Mengen eine toxische Wirkung zeigt, so dass es besonders zuverlässig zur Abtötung von Keimen oder Mikroorganismen verwendet werden kann, wobei es andererseits aber- im Gegensatz beispielsweise zu Silber oder anderen mikrobiziden Grundstoffen - Bestandteil des natürlichen menschlichen Stoffwechsels und daher grundsätzlich für den menschlichen Organismus unschädlich ist.

Das Formteil 1 ist für eine besonders hohe Flexibilität und variable Einsatzmöglichkeiten ausgeführt. Um dies zu ermöglichen, weist der Berührungsbereich 2 an seiner frei zugänglichen und somit berührbaren Oberfläche 4 eine mikrobizide Oberflächenschicht 6 auf. Diese mikrobizide Oberflächenschicht 6 wird durch eine Silikonmatrix 8 gebildet, in die als eigentlich mikrobizides Material Kupfer-Partikel 10 eingebettet sind.

Im Hinblick auf die gewünschte Wirkungsweise, also der gezielten Nutzung der mikrobiziden Eigenschaften des in die Silikonmatrix 8 eingelagerten Kupfers, ist die Oberflächenschicht 6 gezielt auf eine ausreichend hohe Ausschüttung von Kupferionen an den Oberflächenbereich ausgeführt. Wie sich überraschenderweise herausgestellt hat, ist dies auf besonders günstige Weise erreichbar, indem die Kupferpartikel 10 in der Silikonmatrix 8 als so genannte Nano- oder Mikropartikel, also als Partikel mit einer durchschnittlichen Größe im Nano- bzw. Mikrometerbereich, vorliegen. Dementsprechend weisen die in die Matrix 8 eingebetteten Kupfer-Partikel 10 im Ausführungsbeispiel eine mittlere Partikelgröße von etwa 20 - 50 µm, mit einer Standardabweichung von höchstens 20%, auf. In alternativer vorteilhafter Ausgestaltung können die Kupfer-Partikel 10 auch eine Partikelfraktion mit einer Partikelgröße von höchstens 500 nm, vorzugsweise mit einer Partikelgröße von 90 bis 250 nm, umfassen.

Die Kupfer-Partikel 10 können in sich weitgehend homogen und als Vollkörper ausgeführt sein und insbesondere aus reinem oder nahezu reinem Kupfer, ggf. auch aus einer geeignet gewählten Legierung bestehen. Alternativ und bevorzugt ist aber auch ein besonders materialsparender Einsatz des Kupfers denkbar, indem die Kupfer-Partikel 10 ihrerseits als außenseitig mit Kupfer beschichtete Partikel eines Trägermaterials ausgeführt sind. Diese Partikel können beispielsweise außenseitig unter Rückgriff auf die Wirbelschichttechnik mit einer Hülle aus Kupfer beschichtet werden.

Die Wirbelschichttechnik ermöglicht, ähnlich einer Plasmabeschichtung, jedoch speziell für Kleinstpartikel, im industriellen Maßstab zu beschichten. Für die spezielle Anwendung, also insbesondere die Beschichtung mit Kupfer, besonders geeignet und daher besonders bevorzugt sind als Basis- oder Trägermaterial insbesondere Blähton oder auch vergleichsweise leichte Metalle und/oder Metalle, die selbst eine antimikrobielle Wirkung haben. Durch eine derartige Ausgestaltung der Partikel 10 kann zusätzlich zu einem besonders wirksamen Ressourceneinsatz und der dadurch erreichbaren Kostensenkung auch eine verbesserte Oberflächenbeschaffenheit im Endprodukt und/oder auch eine Optimierung des Wirkmechanismus erreicht werden.

Hinsichtlich der Ausgestaltung der Oberflächenschicht 6 sind mehrere als besonders vorteilhaft angesehene Varianten denkbar. Im Ausführungsbeispiel gem. FIG. 1 ist die Oberflächenschicht 6 als auf einen Tragkörper12 aufgebrachte Beschichtung 14 ausgeführt.

Das zur Bildung der Silikonmatrix 8 vorgesehene Material ist gezielt im Hinblick auf eine günstige Verarbeitbarkeit und Applizierbarkeit ausgewählt. Insbesondere ist zur Herstellung des Berührungsbereichs 2 bzw. von dessen Oberflächenschicht 6 nämlich vorgesehen, die die Kupfer-Partikel 10 enthaltende Matrix 8 durch ein Tauchbeschichtungsverfahren oder auch durch ein Verfahren der Spritztechnik (vorzugsweise Umspritzung) aufzubringen. Dementsprechend ist das zur Bildung der Matrix 8 vorgesehene Ausgangsmaterial gezielt unter Berücksichtigung der Auslegungsparameter gewählt, dass unter anderem einerseits die Kupferpartikel 10 zuverlässig und gff. auch gleichmäßig aufgenommen werden, und dass andererseits eine besonders gute Verarbeitbarkeit zur Aufbringung der Beschichtung 14 gegeben ist. Dabei ist vorgesehen, die Kupferpartikel 10 nach geeigneter Vorbereitung, insbesondere nach einem Stabilisierungsschritt, in eine Flüssigkeit einzubringen und in dieser zu homogenisieren und gleichmäßig zu verteilen. Anschließend soll die mit den Partikeln 10 beladene Flüssigkeit im Rahmen einer Tauchbeschichtung auf den Tragkörper 12 aufgebracht werden, und anschließend ist ein Aushärteschritt, ggf. mit Unterstützung durch thermische Behandlung, zur Bildung der eigentlichen Matrix 8 vorgesehen. Im Hinblick auf diese Kriterien ist als das zur Bildung der Matrix 8 vorgesehene Ausgangsmaterial ein Silikonkautschuk oder Silikonelastomer (alternativ Po-ly(organo)siloxane), für bestimmte Anwendungen auch Silikonharz (Polymethylsiloxane oder Polymethylphenylsiloxane) und Fluorsilikone vorgesehen.

Im alternativen Ausführungsbeispiel gem. FIG. 2 ist das Formteil 1' hingegen zumindest in seinem Berührungsbereich 2' als Guß- oder Vollkörper 20 ausgeführt.

Dieser wird durch das ausgehärtete, mit den eingebetteten Kupfer-Partikeln 10 versehene Matrixmaterial gebildet. Im Hinblick auch auf diese Kriterien ist als das zur Bildung der Matrix 8 vorgesehene Ausgangsmaterial ebenfalls ein medizinisches Silikon, vorzugsweise ein HTV-Silikon oder in bestimmten Fällen auch ein RTV-Silikon vorgesehen. Bei der Herstellung ist im Hinblick auf möglichst günstige mikrobizide Eigenschaften der eingebetteten Kupfer-Partikel 10 eine Platinvernetzung vorgesehen. Bei der Herstellung des Vollkörpers 20 kann auf herkömmliche, auf das verwendete Matrixmaterial geeignet abgestimmte Verfahren zurückgegriffen werden.

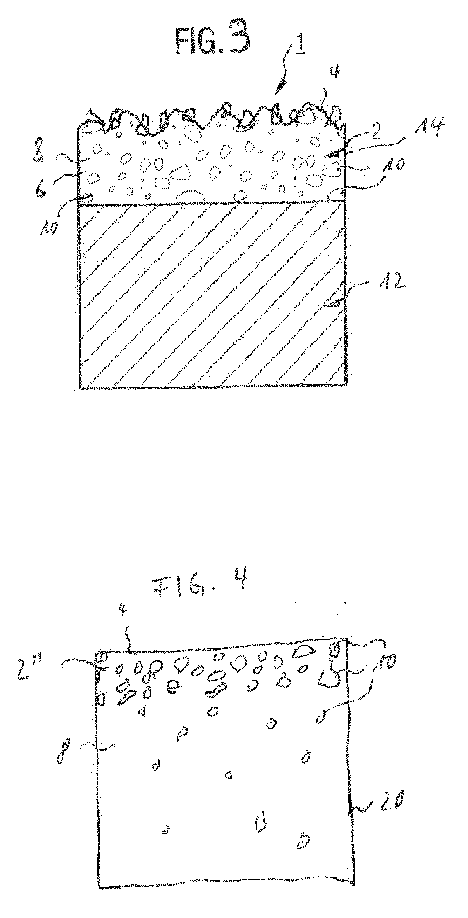

In den in FIG. 1,2 gezeigten Ausführungsbeispielen sind die Kupfer-Partikel 10 im Wesentlichen vollständig in die Matrix 8 eingebettet und von dieser umschlossen. Um die auslegungsbedingt vorgesehene und auch erwünschte ausreichend hohe Ionenausschüttung oder- abgabe noch weiter zu begünstigen, kann der Berührungsbereich 2 des Formteils 1 aber in einer besonders bevorzugten, in FIG. 3 dargestellten Ausführungsform auch derart ausgestaltet sein, dass die Kupfer-Partikel 10 im Oberflächenbereich der Oberflächenschicht 6 zumindest teilweise unbedeckt von der Matrix 8 vorliegen. Damit ist zusätzlich zu einer insgesamt erhöhten lonenausschüttung im frei liegenden Berich der Partikel ein dirketer Kontakt von (Keim-) Zellhüllen mit Kupferatomen möglich, so dass gezielt die Zellhüllen geschwächt oder geschädigt werden können. Zur Herstellung der entsprechenden Oberflächenbereiche ist vorzugsweise ein Aufrauhungs- und/oder Ätzprozeß der Matrix 8 vorgesehen, mit dem das Matrixmaterial oberhalb der eingebundenen Kupfer-Partikel 10 zumindest teilweise entfernt und deren Oberfläche somit zumindest teilweise freigelegt wird. Als besonders zuverlässig und somit besonders bevorzugt hat sich zu diesem Zweck eine Anrauung der Oberfläche durch mechanisches Schleifen erwiesen.

Im Ausführungsbeispiel und besonders bevorzugt beträgt der durch die frei liegenden Kupfer-Partikel 10 gebildete Anteil der Gesamtoberfläche der Oberflächenschicht 6 mindestens 10%.

In den Ausführungsbeispielen gem. FIG. 1 bis 3 ist die Oberflächenschicht 6 mit einer zumindest annähernd räumlich homogenen, gleichmäßigen Verteilung der eingebetteten Kupfer-Partikel 10 ausgeführt, bei der sämtliche Raumbereiche der Oberflächenschicht 6 eine vergleichbare Konzentration an eingebetteten Kupfer-Partikeln 10 aufweisen.

In dem alternativen, ebenfalls als besonders vorteilhaft angesehenen Ausführungsbeispiel gem. FIG. 4 ist die Konzentration der eingebetteten Kupfer-Partikel 10 aber hingegen räumlich inhomogen, wobei im Bereich nahe der Oberfläche 4 eine Überhöhung vorliegt, so dass der überwiegende Anteil der Kupfer-Partikel 10 nah zur Oberfläche 4 des Berührungsbereichs 2" positioniert ist. Ein derartiger, gezielt gewählter Konzentrationsverlauf der Kupfer-Partikel 10 ist beispielsweise erhältlich, indem die Kupfer-Partikel 10 dem zur Bildung der Matrix 8 vorgesehenen Ausgangsmaterial beigemischt und in diesem vorübergehend in Suspension oder Schwebe gehalten werden. Während des Aushärtens des Ausgangsmaterials zur Bildung der Matrix 8 können die Kupfer-Partikel 10 schwerkraftbedingt zu Boden sinken, so dass eine Anreicherung der Partikel im unteren Grenzflächenbereich des entstehenden Gusskörpers erfolgt. Dieser untere Grenzflächenbereich kann sodann zur Bildung des Berührungsbereichs 2" herangezogen werden.

Im Sinne einer ausreichend hohen mechanischen Stabilität ist in sämtlichen vorgenannten Ausführungsbeispielen für das Matrixmaterial, das in der Art eines Bindemittels die mechanische Stabilität unter den Kupfer-Partikeln 10 herstellt, ein geeignet gewählter Volumenanteil an der Oberflächenschicht 6 insgesamt vorgesehen. Um dem Rechnung zu tragen, beträgt im Ausführungsbeispiel der Volumenanteil der Kupfer-Partikel 10 am Gesamtvolumen der Oberflächenschicht 6 zwischen 5% und 40%.

Die Herstellung des Formteils 1 ist für alle Varianten, abgesehen von der Bereitstellung der frei zugänglichen Oberfläche der Kupfer-Partikel 10, und abgesehen von dem Umstand, ob ein Vollkörper 20 oder eine Beschichtung 14 gewünscht ist, mehr oder weniger identisch. Nachfolgend werden daher einige Beispiele zur Herstellung der Beschichtung 14 näher erläutert, die sich hinsichtlich der erzielten Ergebnisse - ggf. unter Rückgriff auf geeignete standardisierte Gussverfahren - problemlos auch auf die Herstellung von Vollkörpern 20 übertragen lassen. Insbesondere hat sich für die Herstellung der Beschichtung 14, unter Anwendung eines Tauchbeschichtungsverfahrens, ein Vorgehen entsprechend der nachfolgend aufgeführten Beispiele als besonders vorteilhaft erwiesen:

### Herstellung der Silikongrundmischung

Als Basis wurde ein HTV Silikon mit einem Shore Wert von 60 und einem Vernetzungskatalysator aus Platin gemischt. Inkl. Test auf sogenannte Katalysatorgifte.

### Homogene Einmischung der Kupfer-Partikel 10:

Die Kupferpartikel wurden der Silikon Grundmasse beigemischt. Um eine ausreichende Homogenität zu gewährleisten, und um eine durch Hitze ausgelöste chemische Reaktion zu verhindern, wurde bevorzugt eine Walzenmischung angewendet. Hierbei wurde das Silikon Kupfer-gemisch für mehrere Stunden über ständig gekühlte Walzen geführt, bis die gewünschte Durchmischung erreicht war.

### Formgebung und Vernetzung des Silikons :

Die Silikon-Kupfermischung wurde in eine Form gegossen und bei ca. 120° C durch die Heizstraße geführt. Temperatur und Zeitraum sowie der gewählte Katalysator haben sich hierbei als entscheidend für die antimikrobielle Wirksamkeit herrausgestellt.

### RTV Silikon:

Es wurde ein 2 Komponenten Silikon mit Platinkatalysator gewählt aufgrund der guten medizinischen Eignung und der geringen Wechselwirkung mit Kupfer

Die Eigenschaften eines beispielhaft hergestellten, mit Kupfer-Partikeln 10 versehenen Silikon-Körpers mit besonders hoher mikrobizider Wirksamkeit sind im Folgenden zusammengestellt:

| Probe # | Härte (°Shore A) | Dichte (g/cm³) | Reißfestigkeit (N/mm²) | Reißdehnung (%) | WRF (N/mm) | RPE (%) | DVR (%) |
|---|---|---|---|---|---|---|---|
| 1 | 46,8 | 2,27 | 5,3 | 497,1 | 14,6 | 44,1 | 84,6 |
| 2 | 68,7 | 2,4 | 5,1 | 367,2 | 15,6 | 35 | 92,9 |

### Bezugszeichenliste

- 1,1': Formteil
- 2,2': Berührungsbereich
- 4: Oberfläche
- 6: Oberflächenschicht
- 8: Matrix
- 10: Kupfer-Partikel
- 12: Tragkörper
- 14: Beschichtung
- 20: Vollkörper

## Patentansprüche

1. Formteil (1,1'), das in einem Berührungsbereich (2,2') eine mikrobizide, aus in eine Silikonmatrix (8) eingebetteten Kupfer-Partikeln (10) gebildete Oberflächenschicht (6) aufweist.

2. Formteil (1,1') nach Anspruch 1, bei dem die Silikonmatrix (8) aus einem Silikonelastomer gebildet ist.

3. Formteil (1,1') nach Anspruch 1 oder 2, bei dem die Oberflächenschicht (6) Teil eines aus Silikon, vorzugsweise einem Silikonelastomer, gebildeten Vollkörpers ist.

4. Formteil (1,1') nach einem der Ansprüche 1 bis 3, bei dem die in die Silikonmatrix (8) eingebetteten Kupfer-Partikel (10) eine Partikelfraktion mit einer Partikelgröße von höchstens 500 nm, vorzugsweise mit einer Partikelgröße von 90 bis 250 nm, umfassen.

5. Formteil (1,1') nach Anspruch 4, bei dem die Partikelfraktion eine mittlere Partikelgröße von etwa 50 µm mit einer Standardabweichung von höchstens 20% aufweist.

6. Formteil (1,1') nach einem der Ansprüche 1 bis 5, bei dem die in die Silikonmatrix (8) eingebetteten Kupfer-Partikel (10) eine Partikelfraktion mit einer Partikelgröße von mindestens 1 µm umfassen.

7. Formteil (1,1') nach einem der vorhergehenden Ansprüche, bei dem die Kupfer-Partikel (10) im Oberflächenbereich der Schicht (6) zumindest teilweise unbedeckt vom Matrixmaterial vorliegen.

8. Formteil (1,1') nach Anspruch 7, bei dem der durch die frei liegenden Kupfer-Partikel (10) gebildete Anteil der Gesamtoberfläche der Oberflächenschicht (6) mindestens 10 % beträgt.

9. Formteil (1,1') nach einem der vorhergehenden Ansprüche, bei dem der Volumenanteil der Kupfer-Partikel (10) am Gesamtvolumen der Oberflächenschicht (6) zwischen 10% und 80% beträgt.

10. Verwendung eines Formteils (1,1') nach einem der vorhergehenden Ansprüche als Dichtungselement, O-Ring,Türdichtungsprofil, Abdichtstreifen beispielsweise für Kühlschränke oder Gefriertruhen, Schlauch, Oberflächenbeschichtung für Ablagen, Medizingeräte, in Abflusssystemen, Tischen oder Sportgeräten, Beatmungsgeräte, Beschichtung von Griffen, Beschichtung von textilem Gewebe, Dichtung in Armaturen oder in Zubehör für Duschköpfe, Schutzhülle für Mobiltelefone, griffiger Überzug für Hanteln und Trainingsgeräte, Schuheinlagen (auch in Verbindung mit anderem Material), Matratzenauflage oder Zusatz dafür, Auflage für Operationstische zur Druckentlastung, Tauchermaske, Taucheranzug.

11. Verfahren zur Herstellung eines Formteils (1,1') nach einem der vorhergehenden Ansprüche, bei dem ein zur Herstellung eines Silikon-Körpers vorgesehenes Ausgangsmaterial durch Einrühren mit Kupfer-Partikeln (10) beladen wird.

12. Verfahren nach Anspruch 11, bei dem die Kupfer-Partikel (10) vor dem Einbringen in das Ausgangsmaterial stabilisiert werden.

13. Verfahren nach Anspruch 12, bei dem die Kupfer-Partikel (10) zur Stabilisierung und Homogenisierung einem Behandlungsschritt unterzogen werden, bei dem sie in einer Mischung aus einem Lösungsmittel, vorzugsweise Ethanol, und Polyvinylpyrrolidon (PVP) mit Ultraschall beaufschlagt werden.
